# EUROPÄISCHE PATENTSCHRIFT

(11) **EP 0 501 267 B1**
(45) Veröffentlichungstag und Bekanntmachung des Hinweises auf die Patenterteilung: **15.04.1998**
(21) Anmeldenummer: 92102587.0
(22) Anmeldetag: 17.02.1992
(51) Int. Cl.: A61K 47/48, A23L 1/275, A61K 7/42

(54) **Einschlussverbindungen von Apocarotinal oder Lycopin in einem Cyclodextrin**
Apocarotin or lycopin cyclodextrin clathrate
Composés d'inclusion d'apocarotinal ou de lycopin dans une cyclodextrine

(30) Priorität: 25.02.1991 CH 556/91
(43) Veröffentlichungstag der Anmeldung: 02.09.1992
(73) Patentinhaber: F. HOFFMANN-LA ROCHE AG, 4002 Basel (CH)
(72) Erfinder: Leuenberger, Bruno, CH-4057 Basel (CH); Stoller, Hansjörg, CH-4153 Reinach (CH)
(74) Vertreter: Cottong, Norbert A.

(56) Entgegenhaltungen:
- EP-A- 0 392 608
- WO-A-82/00251
- WO-A-89/10739
- DE-B- 1 050 167
- PATENT ABSTRACTS OF JAPAN vol. 12, no. 155 (C-494)(3002) 12. Mai 1988 & JP-A-62 267 261 ( ICHIMARU FUARUKOSU K.K. ) 19. November 1987

## Beschreibung

Die Erfindung betrifft in polaren Lösungsmitteln lösliche Einschlussverbindungen von Cyclodextrinen mit Apocarotinal oder Lycopin sowie Verfahren zu deren Herstellung.

Cyclodextrine bilden mit einer Vielzahl von Stoffen Einschlussverbindungen, wodurch es beispielsweise möglich wird, unlösliche Verbindungen als Einschlussverbindungen in einem geeigneten Lösungsmittel zu lösen. Besonders interessant wäre die Bildung von löslichen Einschlussverbindungen mit Vitaminen und insbesondere mit Carotinoiden, da diese Verbindungen als solche im allgemeinen in polaren Lösungsmitteln, insbesondere Wasser, unlöslich sind und es für die meisten Anwendungen wünschenswert ist, dass diese Verbindungen in einer löslichen Form vorliegen. Dies ist insbesondere von Bedeutung, um eine erhöhte Bioverfügbarkeit der Wirkstoffe zu erreichen. Ferner ist im Falle von Carotinoiden die dadurch erzielte Farbkraft von grossem Interesse für Färbungszwecke bei Lebensmitteln.

Bisher wurden verschiedene Verfahren bekannt, die jedoch alle auf der Herstellung einer Suspension oder Emulsion beruhen. Die Carotinoide werden hierzu beispielsweise in einer Oelphase und/oder in einem organischen Lösungsmittel gelöst und mit Wasser zu einer Emulsion verarbeitet.

In einem anderen Verfahren wird ein Carotinoid in einem Oel gemahlen und die gebildete Suspension anschliessend in Wasser einemulgiert.

Diese Formulierungen haben jedoch den Nachteil, dass sie in Wasser eine Trübung aufweisen, da die Suspension oder Emulsion aus Teilchen oder lichtstreuenden Tröpfchen besteht. Weiterhin sind derartige Suspensionen oder Emulsionen der Carotinoide nicht dauerhaft stabil. Ferner werden die Carotinoide durch Einwirkung von Wärme und Licht leicht zerstört. In derartigen Suspensionen und Emulsionen liegen die Wirkstoffe in der Regel als Partikel der Grösse 0,1 bis 10 µm vor.

Eine grundsätzlich andere Methode wird in der japanischen Offenlegungsschrift Kokai 26 72 61 beschrieben. Hier wird β-Carotin mit einem Cyclodextrin zusammengegeben. Der Nachteil dieser Methode ist jedoch darin zu sehen, dass hierbei keine Lösungen erhalten werden, sondern Suspensionen gebildet werden, die sich beim Stehenlassen wieder separieren.

Die PCT-Publication Nr. Wo 82/00251 offenbart wasserlösliche Cyclodextrin-Komplexe von freien und, vor allem, derivatisierten Retinoiden, welche verbesserte hydrophile Eigenschaften auf Kosten der fettlöslichen Eigenschaften aufweisen. Cyclodextrin-Komplexe von anderen Verbindungen als Retinoiden bzw. Retinoid-Derivaten sind in dieser Patentpublikation gar nicht erwähnt und auch nicht angedeutet.

Es wurden nun Einschlussverbindungen der Polyene Apocarotinal und Lycopin in Cyclodextrin hergestellt, welche die Nachteile des Standes der Technik nicht aufweisen.

Gegenstand der Erfindung sind in einem polaren Lösungsmittel lösliche Einschlussverbindungen des Polyens Apocarotinal oder Lycopin in einem Cyclodextrin und deren Herstellung.

Als Cyclodextrin wird vorzugsweise α-Cyclodextrin, Methyl-β-cyclodextrin oder Hydroxypropyl-β-cyclodextrin verwendet.

Die neuen Einschlussverbindungen werden erfindungsgemäss dadurch erhalten, dass man ein Cyclodextrin mit Apocarotinal und/oder Lycopin sowie mit einem polaren Lösungsmittel zusammenbringt. Dabei kann man so vorgehen, dass man
a) ein Cyclodextrin in einem polaren Lösungsmittel löst und diese Lösung mit Apocarotinal oder Lycopin versetzt, oder
b) einerseits Apocarotinal oder Lycopin in einem organischen Lösungsmittel löst und andererseits ein Cyclodextrin in einem polaren Lösungsmittel löst und beide Lösungen zusammengibt, oder
c) festes Apocarotinal oder festes Lycopin in Gegenwart geringer Wassermengen mit einem Cyclodextrin intensiv durchmischt und diese Mischung anschliessend mit einem polaren Lösungsmittel zusammenbringt.

Als bevorzugte Ausführungsform wird Verfahren a) verwendet.

Für die Herstellung von Einschlussverbindungen, welche Apocarotinal enthalten, werden vorzugsweise β-Cyclodextrine, insbesondere Methyl-β-cyclodextrin oder Hydroxypropyl-β-cyclodextrin verwendet, wobei Methyl-β-cyclodextrin besonders bevorzugt ist. Für die Herstellung von Einschlussverbindungen, welche Lycopin enthalten, wird vorzugsweise α-Cyclodextrin verwendet.

Als polares Lösungsmittel wird bevorzugt Wasser oder ein Niederalkylalkohol oder ein Gemisch von Wasser und einem Niederalkylalkohol eingesetzt. Im Falle des Gemisches beträgt das Volumenverhältnis zwischen Wasser und Niederalkylalkohol beträgt zweckmässigerweise etwa 1:30 bis etwa 10:1.

Als Niederalkylgruppen der obenerwähnten Niederalkylalkohole kommen insbesondere Alkylgruppen, die bis zu 6 Kohlenstoffatome enthalten und geradkettig oder verzweigt sind. Der Niederalkylalkohol ist vorzugsweise Methanol, Aethanol oder n-Propanol, insbesondere Methanol oder Aethanol.

Insbesondere eignet sich jedoch Wasser als polares Lösungsmittel.

Das Cyclodextrin kann also, wie oben unter a) beschrieben, zweckmässigerweisezu etwa der 8-fachen bis 10-fachen Menge destillierten Wassers, Niederalkylalkohol oder der Mischung aus Wasser und Niederalkylalkohol gegeben und bei einer Temperatur von etwa 5°C bis 90°C gelöst werden. Bevorzugt wird in einem Temperaturbereich von etwa 20°C bis 60°C gearbeitet. Zu dieser Lösung wird das Apocarotinal oder das Lycopin zugegeben, wobei die Menge des eingesetzten Polyens zweckmässigerwqeise etwa 1/10 bis 1/10.000 der Menge des eingesetzten Cyclodextrins entspricht. Bevorzugt beträgt das Gewichtsverhältnis zwischen Polyen und Cyclodextrin etwa 1:10 bis etwa 1:200.

Das Polyen kann auch gemäss b) zweckmässigerweise als etwa 8-12%ige Lösung, insbesondere als etwa 10%ige Lösung, in einem organischen Lösungsmittel der Lösung vom Cyclodextrin in dem polaren Lösungsmittel, insbesondere Wasser, zugegeben werden, wobei als organisches Lösungsmittel insbesondere Chloroform oder Hexan verwendet wird. Das organische Lösungsmittel wird in einem zweiten Schritt, z.B. durch Destillation, entfernt.

Alternativ können die Polyene Apocarotinal und Lycopin gemäss c) zweckmässigerweise in fester Form zunächst in Gegenwart geringer Mengen Wasser mit etwa der 10-fachen bis 12-fachen Menge Cyclodextrin intensiv durchmischt werden. Anschliessend wird diese Mischung mit einem polaren Lösungsmittel zusammengebracht und eventuell vorhandene feste Bestandteile nach üblichen Methoden, beispielsweise durch Filtrieren, entfernt.

Der Temperaturbereich ist nicht kritisch. Insbesondere wird in einem Temperaturbereich von etwa 5°C bis etwa 90°C gearbeitet, wobei ein Temperaturbereich von etwa 20°C bis etwa 60°C besonders bevorzugt ist. Die Mischung wird für einen Zeitraum von etwa 20 Minuten bis etwa 70 Minuten weitergerührt, wobei eventuell vorhandenes Lösungsmittel verdampft, und anschliessend filtriert, um eventuell vorhandene feste Bestandteile von der Lösung zu trennen. Das Filtrieren kann bei Raumtemperatur oder erhöhter Temperatur durchgeführt werden. Bevorzugt wird nach Abkühlen der Lösung auf Raumtemperatur filtriert.

Die aus Apocarotinal und Methyl-β-cyclodextrin gebildete Einschlussverbindung kann durch Verdampfen des Lösungsmittels in fester Form isoliert und anschliessend auch wieder gelöst werden. Dieser Vorgang ist beliebig oft wiederholbar, ohne dass merkliche Veränderungen auftreten.

Als Vorteile der erfindungsgemässen Einschlusskomplexe gegenüber den bisher bekannten Cyclodextrin/Polyen-Formulierungen sei besonders hervorgehoben, dass diese neuen Komplexe
- echte Lösungen in Wasser oder Wasser/Alkohol-Gemischen bilden, was die Herstellung stabiler und trübungsfreier Lösungen erlaubt und durch das molekulardisperse Vorliegen von Apocarotinal bzw. Lycopin eine wesentlich verbesserte Bioverfügbarkeit bedingt,
- einen deutlich vergrösserten Farbbereich haben,
- die Stabilität der eingeschlossenen Polyene Apocarotinal und Lycopin gegenüber Wärme und Licht wesentlich erhöht wird.

Die Erfindung wird durch folgende Beispiele weiter veranschaulicht:

### Beispiel 1

5.0 g Methyl-β-cyclodextrin (Wacker Chemie, Deutschland; statisch methyliertes β-Cyclodextrin mit ca. 1,8 CH₃-Gruppen pro Anhydroglucose-Einheit) werden bei einer Temperatur von 60°C in 40 ml Wasser gelöst. Zu dieser Lösung werden 0,5 g kristallines Apocarotinal zugegeben. Die Mischung wird auf 55°C erwärmt und bei dieser Temperatur 20 Minuten weitergerührt. Nach Abkühlen auf Raumtemperatur werden die festen Bestandteile von der tiefroten Lösung abfiltriert.

Die spektralphotometrische Messung dieser Lösung ergibt pro ml Lösung einen Gehalt an Apocarotinal von 0,16 mg.

Unter Verwendung eines Laborsprühtrockner der Firma Büchi (Schweiz) wird die wässrige Lösung des Apocarotinal-Cyclodextrin-Komplexes in ein festes Pulver übergeführt. Das erhaltene Pulver zeigt gute Kaltwasserlöslichkeit und ergibt bei Zugabe von Wasser wieder die wässirge Lösung.

Die Lösung des Apocarotinal-Cyclodextrin-Komplexes in Wasser und eine Lösung gleicher Konzentration von Apocarotinal in Hexan werden 10 Tage bei Tageslicht stehen gelassen. Die Apocarotinal-Hexan-Lösung verbleicht zu einer hell-orangen Lösung, während die wässrige Lösung des Apocarotinalcyclodextrin-Komplexes keine Veränderung der Farbe aufweist.

### Beispiel 2

5,0 g Hydroxypropyl-β-cyclodextrin (Roquette Frères, Frankreich; ca. 0,6 CH₃CH(CH₃)O-Gruppen pro Anhydroglucose-Einheit) werden mit 0,3 g Apocarotinal vermischt und mit ca. 1 ml Wasser befeuchtet. Das Gemisch wird bei Raumtemperatur während 3 Stunden geknetet, anschliessend bei Raumtemperatur in 40 ml Wasser gegeben und durch Filtrieren von festen Bestandteilen befreit. Die resultierende klare, dunkelrote Lösung enthält pro ml Lösung 3 µg Apocarotinal.

### Beispiel 3

5,0 g α-Cyclodextrin (Wacker Chemie, Deutschland) werden bei einer Temperatur von 60°C in 40 ml Wasser gelöst. Zu diesem Gemisch werden 0,3 g Lycopin, gelöst in 4 ml Chloroform, zugegeben. Diese Suspension wird auf 60°C erwärmt und 20 Minuten weitergerührt, wobei das Chloroform verdampft. Nach dem Abkühlen der Lösung auf Raumtemperatur werden alle festen Bestandteile abfiltriert. Die resultierende dunkelgelbe Lösung enthält pro ml 2 µg Lycopin.

## Patentansprüche

1. Einschlussverbindungen des Polyens Apocarotinal oder Lycopin in einem Cyclodextrin, welche in einem polaren Lösungsmittel löslich sind.

2. Einschlussverbindungen nach Anspruch 1, dadurch gekennzeichnet, dass als Cyclodextrin α-Cyclodextrin, Methy-β-cyclodextrin oder Hydroxypropyl-β-cyclodextrin verwendet wird.

3. Einschlussverbindungen nach Anspruch 2, dadurch gekennzeichnet, dass als Cyclodextrin α-Cyclodextrin und als Polyen Lycopin verwendet wird.

4. Einschlussverbindungen nach Anspruch 2, dadurch gekennzeichnet, dass als Cyclodextrin Methyl-β-cyclodextrin oder Hydroxypropyl-β-cyclodextrin und als Polyen Apocarotinal verwendet wird.

5. Einschlussverbindungen nach einem der Ansprüche 1-4, dadurch gekennzeichnet, dass diese in Wasser, Niederalkylalkohol oder einem Gemisch aus Wasser und Niederalkylalkohol löslich sind.

6. Einschlussverbindungen nach Anspruch 5, dadurch gekennzeichnet, dass im Falle des Gemisches das Volumenverhältnis zwischen Wasser und Niederalkylalkohol etwa 1:30 bis etwa 10:1 beträgt.

7. Einschlussverbindungen nach Anspruch 5 oder 6, dadurch gekennzeichnet, dass als Niederalkylalkohol Methanol oder Ethanol verwendet wird.

8. Verfahren zur Herstellung von Einschlussverbindungen nach einem der Ansprüche 1-7, dadurch gekennzeichnet, dass man ein Cyclodextrin mit Apocarotinal und/oder Lycopin sowie mit einem polaren Lösungsmittel zusammenbringt.

9. Verfahren nach Anspruch 8, dadurch gekennzeichnet, dass das Cyclodextrin in einem Temperaturbereich von etwa 5°C bis etwa 90°C in etwa der 8-fachen bis 10-fachen Menge destillierten Wassers, Niederalkylalkohol oder einer Mischung aus Wasser und Niederalkylalkohol gelöst wird und danach das Apocarotinal oder das Lycopin dieser Mischung zugegeben wird, wobei die Menge des eingesetzten Polyens Apocarotinal oder Lycopin etwa 1/10 bis 1/10.000 der Menge des eingesetzten Cyclodextrins entspricht.

10. Verfahren nach Anspruch 8, dadurch gekennzeichnet, dass das Polyen Apocarotinal oder Lycopin als etwa 8-12%ige Lösung in einem organischen Lösungsmittel der Lösung vom Cyclodextrin in einem polaren Lösungsmittel zugegeben wird.

## Claims

1. Inclusion complexes of the polyene apocarotenal or lycopene in a cyclodextrin, which are soluble in a polar solvent.

2. Inclusion complexes according to claim 1, wherein the cyclodextrin is α-cyclodextrin, methyl β-cyclodextrin or hydroxypropyl β-cyclodextrin.

3. Inclusion complexes according to claim 2, wherein the cyclodextrin is α-cyclodextrin and the polyene is lycopene.

4. Inclusion complexes according to claim 2, wherein the cyclodextrin is methyl β-cyclodextrin or hydroxypropyl β-cyclodextrin and the polyene is apocarotenal.

5. Inclusion complexes according to any one of claims 1-4, which are soluble in water, a lower alkyl alcohol or a mixture of water and a lower alkyl alcohol.

6. Inclusion complexes according to claim 5, wherein in the case of a mixture the volume ratio between water and lower alkyl alcohol is about 1:30 to about 10:1.

7. Inclusion complexes according to claim 5 or 6, wherein the lower alkyl alcohol is methanol or ethanol.

8. A process for the manufacture of inclusion complexes according to any one of claims 1-7, which process comprises bringing a cyclodextrin together with apocarotenal and/or lycopene and with a polar solvent.

9. A process according to claim 8, wherein the cyclodextrin is dissolved in a temperature range of about 5°C to about 90°C in about an 8-fold to 10-fold amount of distilled water, a lower alkyl alcohol or a mixture of water and a lower alkyl alcohol and thereafter the apocarotenal or the lycopene is added to this mixture, the amount of the polyene apocarotenal or lycopene used corresponding to about 1/10 to 1/10,000 of the amount of cyclodextrin used.

10. A process according to claim 8, wherein the polyene apocarotenal or lycopene as an about 8-12% solution in an organic solvent is added to a solution of the cyclodextrin in a polar solvent.

## Revendications

1. Composés d'inclusion du polyène apocaroténal ou lycopène dans une cyclodextrine, qui sont solubles dans un solvant polaire.

2. Composés d'inclusion selon revendication 1, caractérisés en ce que l'on a utilisé en tant que cyclodextrine l'alpha-clodextrine, la méthyl-bêta-cyclodextrine ou l'hydroxypropyl-bêta-cyclodextrine.

3. Composés d'inclusion selon revendication 2, caractérisés en ce que l'on a utilisé en tant que cyclodextrine l'alpha-cyclodextrine et en tant que polyène le lycopène.

4. Composés d'inclusion selon revendication 2, caractérisés en ce que l'on a utilisé en tant que cyclodextrine la méthyl-bêta-cyclodextrine ou l'hydroxypropyl-bêta-cyclodextrine et en tant que polyène l'apocaroténal.

5. Composés d'inclusion selon une des revendications 1 à 4, caractérisés en ce qu'ils sont solubles dans l'eau, un alcanol inférieur ou un mélange d'eau et d'un alcanol inférieur.

6. Composés d'inclusion selon revendication 5, caractérisés en ce que dans le cas du mélange, le rapport en volume entre l'eau et l'alcanol inférieur va d'environ 1 : 30 à 10: 1.

7. Composés d'inclusion selon revendication 5 ou 6, caractérisés en ce que l'alcanol inférieur est le méthanol ou l'éthanol.

8. Procédé de préparation des composés d'inclusion selon une des revendications 1 à 7, caractérisé en ce que l'on mélange une cyclodextrine avec l'apocaroténal et/ou le lycopène et un solvant polaire.

9. Procédé selon la revendication 8, caractérisé en ce que l'on dissout la cyclodextrine dans l'intervalle de température d'environ 5 à 90° C dans environ 8 à 10 fois son poids d'eau distillée, d'un alcanol inférieur ou d'un mélange d'eau et d'un alcanol inférieur puis on ajoute l'apocaroténal ou le lycopène au mélange, la quantité du polyène mise en oeuvre, l'apocaroténal ou lycopène, représentant environ 1/10 à 1/10 000 de la quantité de cyclodextrine mise en oeuvre.

10. Procédé selon revendication 8, caractérisé en ce que l'on ajoute le polyène, apocaroténal ou lycopène, à l'état de solution à une concentration d'environ 8 à 12 % dans un solvant organique à la solution de la cyclodextrine dans un solvant polaire.
